# FASCICULE DE BREVET EUROPEEN

(11) **EP 2 401 925 B1**
(45) Date de publication et mention de la délivrance du brevet: **07.05.2014**
(21) Numéro de dépôt: 10305700.6
(22) Date de dépôt: 29.06.2010
(51) Int. Cl.: A23L 1/09, A23L 1/30, A61K 8/73, C08B 30/18, C08L 3/02

(54) **PRODUIT PREBIOTIQUE ET PROCEDE DE FABRICATION**
Präbiotisches Produkt und sein Herstellungsverfahren
Prebiotic product and manufacturing method

(43) Date de publication de la demande: 04.01.2012
(73) Titulaire: Rosebud AG, 6300 Zug (CH)
(72) Inventeur: Allemand, Brigitte, 81500 Marzens (FR); Pousset, Florence, 33320 Eysines (FR)
(74) Mandataire: Hirsch & Associés

(56) Documents cités:
- WO-A1-02/062363
- WO-A1-2007/125558
- WO-A1-2008/091756
- WO-A1-2010/079444
- DE-U1- 20 202 562
- DATABASE GNPD [Online] Mintel; avril 2009 (2009-04), Anonymous: "Banana flavor muffin baking mix", XP002613972, Database accession no. 1075632
- DATABASE GNPD [Online] Mintel; août 2005 (2005-08), Anonymous: "Whole body superfood dietary supplement", XP002613973, Database accession no. 10227537
- DATABASE WPI Week 201002 Thomson Scientific, London, GB; AN 2009-S32778 XP002613974, & CN 101 589 829 A (JIANGXI HESHANYUAN INT FOODS IND CO LTD) 2 décembre 2009 (2009-12-02)

## Description

### DOMAINE DE L'INVENTION

La présente invention concerne un produit prébiotique ainsi que son procédé de fabrication. L'invention concerne également une composition symbiotique comprenant ledit produit prébiotique en association avec des micro-organismes probiotiques.

### ARRIERE-PLAN TECHNIQUE

On connaît l'effet bénéfique sur la santé humaine et animale de l'ingestion de prébiotiques, substances alimentaires ou diététiques généralement composées de polysaccharides et oligosaccharides essentiellement non amidonnés et non digestibles ou d'alcools de sucres non dégradés et non absorbés dans la partie supérieure du tractus digestif, et qui stimulent de façon sélective, dans l'intestin, la croissance et l'activité d'un ou d'un nombre limité de micro-organismes vivants et bénéfiques pour l'hôte (Gibson et al., J. Nutr., 1995, 125(6):1401-12, Dietary modulation of the human colonic microbiota: introducing the concept of Prebiotics). Les prébiotiques sont caractérisés par une valeur calorique faible de l'ordre de 1,5 kcal/g.

Les prébiotiques favorisent la croissance et le métabolisme des bactéries commensales bénéfiques du colon (essentiellement des lactobacilles et bifidobactéries), au détriment des bactéries pathogènes, ce qui entraîne un rééquilibrage de la composition de la flore intestinale (Cummings JH, Macfarlane GT. Br J Nutr. mai 2002, 87 suppl 2 : S145-51, Gastrointestinal effects of prebiotics*).* Les prébiotiques ont des propriétés immunomodulatrices locales, au niveau de la muqueuse intestinale: ils augmentent la production des immunoglobulines, des cytokines et des lymphocytes T. Ils modifient l'épaisseur et la composition de la muqueuse épithéliale du colon en augmentant le nombre de cellules épithéliales calciformes et en favorisant l'apparition de mucines plus acides caractéristiques d'une muqueuse intestinale stabilisée. Ainsi, une flore intestinale stabilisée contribue à la prévention des infections, des processus oxydatifs et surtout, améliore la fonction d'absorption de l'intestin. En outre, les prébiotiques participent indirectement, via la flore commensale, à l'absorption de minéraux comme le fer, le calcium, le magnésium et le zinc, reconnus pour leurs effets bénéfiques sur la santé. Des études montrent que la présence de prébiotiques, tels que les oligofructoses, augmente de façon significative l'absorption du magnésium et l'accrétion du phosphore dans le fémur; de plus, l'ingestion combinée d'oligofructoses et de calcium, à un taux élevé, est considérée comme le régime alimentaire le plus favorable, en particulier pour la solidité des os et la diminution du risque de fractures. Il est connu depuis peu que les prébiotiques ont un site d'action plus large que le système gastro-intestinal, à savoir la peau, le système urinaire, le système respiratoire et le système génital féminin.

Les prébiotiques (en particulier les fructo-oligosaccharides alimentaires) n'ont pas un effet intrinsèque, par eux-mêmes, sur l'épithélium intestinal mais un effet via la flore intestinale (bactéries commensales) et les probiotiques (micro-organismes ayant un effet bénéfique sur la santé lorsqu'ils sont administrés à un hôte humain ou animal); les prébiotiques améliorent la viabilité et favorisent la prolifération et le métabolisme *in vivo* des probiotiques.

Des associations de prébiotiques et de, probiotiques communément appelées symbiotiques, sont utilisées dans l'alimentation ou la thérapie, humaine ou animale.

Les prébiotiques représentent une source d'énergie sélective des probiotiques, en particulier dans les compositions symbiotiques. Un symbiotique exerce à la fois une activité prébiotique et probiotique. Les symbiotiques stimulent le système immunitaire local associé à l'épithélium intestinal, et le système immunitaire systémique en particulier en diminuant la production de cytokines inflammatoires (IL-1, TNF-α) ce qui diminue le risque d'infections. Les symbiotiques augmentent le nombre et la diversité des bactéries fécales chez l'homme, en particulier les lactobacilles et bifidobactéries, augmentent la fréquence des selles chez les patients avec un syndrome du colon irritable et souffrant de constipation, et diminuent les douleurs abdominales et les ballonnements. Les symbiotiques ont un effet protecteur contre la formation des tumeurs en modifiant le métabolisme des bactéries intestinales, ils induisent une diminution de la concentration en β-glucuronidase et ammoniaque du contenu intestinal, diminuent l'incidence de la formation de cryptes aberrantes considérées comme prédictive d'une tumeur.

Cependant, l'effet d'un symbiotique est plus important que celui d'un prébiotique ou d'un probiotique seul. Leurs effets combinés peuvent être additifs ou synergiques et contribuent à une meilleure efficacité de l'activité fonctionnelle intestinale telle qu'une amélioration du processus d'absorption des nutriments, vitamines, minéraux, amino-acides et oligo-éléments. Le document WO 2007/125558 décrit par exemple de telles compositions symbiotiques.

Le document XP002613972 divulgue une préparation pour muffins au goût de banane, laquelle est décrite comme un produit prébiotique et une source de fibre. Cette préparation comprend des polyosides que sont l'inuline et la maltodextrine, ainsi que de la mélasse conventionnelle de cuisine.

Toutefois, les compositions prébiotiques et symbiotiques proposées dans l'état de la technique ne sont pas complètement satisfaisantes.

En particulier, sur le plan nutritionnel, on utilise uniquement des prébiotiques non digestibles (de par leur composition quasi-exclusive en fibres). Aussi, ces prébiotiques (et les symbiotiques correspondants) ont principalement une activité nutritionnelle directe sur la composition et le métabolisme de la flore intestinale, mais une activité nutritionnelle limitée sur l'hôte. Dans les compositions symbiotiques, les prébiotiques sont ainsi parfois décrits comme simples transporteurs de substances bioactives, naturelles ou non.

Par ailleurs, les compositions symbiotiques connues ne sont pas, en raison de leur procédé d'obtention, des formulations homogènes. De ce fait, les prébiotiques sont généralement administrés avec les probiotiques, simultanément par mélange extemporané ou en même temps (par des voies semblables ou différentes) ou séquentiellement.

Il existe donc un besoin de fournir un produit prébiotique présentant à la fois de bonnes qualités nutritionnelles à l'hôte, et pouvant servir de support efficace et souple d'utilisation pour fabriquer des compositions symbiotiques. Parallèlement, il existe un besoin de fournir une composition symbiotique avec une formulation homogène (pour une utilisation variée et étendue), présentant de bonnes qualités nutritionnelles à l'hôte et garantissant une bonne efficacité des micro-organismes probiotiques.

### RESUME DE L'INVENTION

L'invention concerne en premier lieu un produit prébiotique comprenant:
- de la mélasse séchée, en mélange avec
- une composition de polyosides,
dans laquelle la composition de polyosides présente une teneur en fibres supérieure ou égale à 80%.

Selon un mode de réalisation, la composition de polyosides est une composition de dextrine ou de maltodextrine, de préférence une composition de dextrine dérivée de l'amidon de maïs.

Selon un mode de réalisation, le produit prébiotique comprend une proportion massique totale d'eau inférieure ou égale à 6%, de préférence inférieure ou égale à 5%, de manière plus particulièrement préférée inférieure à 4%.

Selon un mode de réalisation, la composition de polyosides présente :
- une teneur en monosaccharides et en disaccharides inférieure ou égale à 5%, de préférence inférieure ou égale à 2%, de manière plus particulièrement préférée inférieure ou égale à 1% et idéalement inférieure ou égale à 0,5% ; et / ou
- une masse moléculaire moyenne en poids de 4000 à 7000 daltons, de préférence de 4500 à 6000 daltons et de manière plus particulièrement préférée de 4800 à 5200 daltons ; et / ou
- une viscosité, à 60% dans de l'eau à 20°C, de 1000 à 2500 mPa.s, de préférence de 1250 à 2000 mPa.s et de manière plus particulièrement préférée de 1400 à 1600 mPa.s ; et / ou
- de 45 à 55% de liaisons glycosidiques (1,4), de 25 à 35% de liaisons glycosidiques (1,6), de 5 à 15% de liaisons glycosidiques (1,2) et de 5 à 15% de liaisons glycosidiques (1,3).

Selon un mode de réalisation, le produit prébiotique comprend, en proportions massiques : de 50 à 80% de la composition de polyosides et de 20 à 50% de mélasse séchée, de préférence de 60 à 70% de la composition de polyosides et de 30 à 40% de mélasse séchée.

Selon un mode de réalisation, la mélasse est choisie parmi les mélasses de canne, de betterave, de fruits, de céréales et leurs mélanges, et de préférence parmi les mélasses de canne, de betterave et leurs mélanges, et de manière plus particulièrement préférée est une mélasse de canne.

L'invention concerne également une composition symbiotique comprenant le produit prébiotique ci-dessus, en mélange avec une composition de micro-organismes probiotiques, de préférence des micro-organismes vivants, déshydratés et encapsulés.

Selon un mode de réalisation, la composition symbiotique comprend en proportions massiques :
- de 10 à 50% de mélasse séchée, de préférence de 15 à 45%, de manière plus particulièrement préférée de 20 à 40% ;
- de 15 à 70% de la composition de polyosides, de préférence de 20 à 65%, de manière plus particulièrement préférée de 35 à 60% ; et
- de 4 à 50% de la composition de micro-organismes probiotiques, de préférence de 10 à 40%, de manière plus particulièrement préférée de 20 à 30% ;
et / ou comprend une quantité de micro-organismes probiotiques vivants d'au moins 10⁵ cfu/g, de préférence au moins 10⁷ cfu/g, idéalement au moins 5×10⁷ cfu/g.

Selon un mode de réalisation, les micro-organismes probiotiques sont choisis parmi les bactéries ou levures des genres *Bifidobacterium, Lactobacillus, Lactococcus, Streptococcus, Pediococcus, Enterococcus, Propionibacterium, Saccharomyces, Kluyveromyces* et leurs mélanges, et de préférence parmi les espèces *Bifidobacterium longum, Lactobacillus casei, Lactobacillus rhamnosus, Lactobacillus helveticus* et *Saccharomyces torula.*

L'invention concerne en outre un produit alimentaire ou diététique comprenant le produit prébiotique ci-dessus, ou la composition symbiotique ci-dessus, par exemple sous forme de barre énergétique, de poudre à diluer, de gélules, de comprimés; produit déshydraté.

L'invention concerne en outre un produit cosmétique comprenant le produit prébiotique ci-dessus, ou la composition symbiotique ci-dessus, par exemple sous forme de pommade, crème, lotions, gels, émulsions, shampoing, cire, masque, mousse à raser, crème dépilatoire, capsule ou gélule.

L'invention concerne en outre un médicament comprenant le produit prébiotique ci-dessus, ou la composition symbiotique ci-dessus, par exemple sous forme de comprimé, tablette à croquer, gélule, capsule, poudre ou granules.

L'invention concerne en outre un procédé de fabrication du produit prébiotique ci-dessus, comprenant une étape de mélange de mélasse avec la composition de polyosides pour obtenir un pré-mélange, puis une étape de séchage du pré-mélange.

L'invention concerne en outre un procédé de fabrication de la composition symbiotique ci-dessus, comprenant :
- une étape de mélange de mélasse avec la composition de polyosides pour obtenir un pré-mélange, puis une étape de séchage du pré-mélange, puis une étape de mélange du pré-mélange séché avec la composition de micro-organismes probiotiques ; ou
- une étape de mélange de mélasse avec la composition de polyosides et la composition de micro-organismes probiotiques pour obtenir un pré-mélange, puis une étape de séchage du pré-mélange.

Selon un mode de réalisation, l'étape de séchage est effectuée au moyen d'une étuve sous vide, par lyophilisation, par rayonnement micro-ondes, au moyen d'un lit fluidisé ou par atomisation, et de préférence par atomisation ; et / ou dans lequel le taux de matière sèche du pré-mélange avant séchage est diminué par ajout d'eau, de préférence jusqu'à une valeur de 30 à 60%, de manière plus particulièrement préférée de 40 à 50% en masse.

La présente invention permet de surmonter les inconvénients de l'état de la technique. Elle fournit plus particulièrement un produit prébiotique présentant de meilleures qualités nutritionnelles et galéniques que les produits prébiotiques de l'état de la technique, ainsi qu'une composition symbiotique homogène, présentant également de meilleures qualités nutritionnelles et galéniques que les compositions symbiotiques de l'état de la technique.

Ceci est accompli grâce à l'utilisation d'un mélange de mélasse séchée et d'une composition de polyosides avec une teneur en fibres supérieure ou égale à 80%, en tant que prébiotique. Ce prébiotique est avantageusement combiné avec des micro-organismes probiotiques pour fournir une composition symbiotique.

La mélasse est un ingrédient extrêmement riche en éléments d'intérêt nutritionnel, tels que sucres en particulier à faible index glycémique compris entre 25 et 28 (index glycémique mesuré *in vitro* selon la méthode Englyst), fibres non digestibles, minéraux, oligo-éléments, vitamines et acides aminés. La mélasse étant sous forme liquide et de forte densité, il ne serait pas approprié de fabriquer un produit prébiotique comprenant directement la composition de polyosides en mélange avec la mélasse, car un tel produit ne pourrait être formulé correctement et en particulier ne pourrait être combiné avec des micro-organismes probiotiques (ces derniers ne survivraient pas, du fait de la pression osmotique).

Selon certains modes de réalisation particuliers, l'invention présente également une, ou de préférence plusieurs, des caractéristiques avantageuses énumérées ci-dessous :
- La mélasse peut remplir à la fois une fonction de prébiotique en servant de support de croissance aux probiotiques et en stimulant leur activité, une fois la composition ingérée, et directement une fonction de supplément diététique pour l'homme ou l'animal ou, pharmaceutique pour l'homme ou l'animal (applications vétérinaires).
- La mélasse fournit directement une grande variété d'éléments d'intérêt nutritionnel, tels que sucres en particulier à faible index glycémique compris entre 25 et 28 (index glycémique mesuré *in vitro* selon la méthode Englyst), fibres, minéraux, vitamines, oligo-éléments, et acides aminés essentiels et non-essentiels, sans qu'il soit nécessaire d'ajouter séparément et individuellement ces éléments à la composition. Ainsi, l'invention est économique.
- La mélasse transformée selon l'invention, permet une grande variété de mise en oeuvre des mélanges symbiotiques, ouvrant à des utilisations et des modes d'administration divers, pour l'homme et l'animal.
- L'invention prévoit d'utiliser de préférence les micro-organismes probiotiques sous forme déshydratée et encapsulée afin de les protéger. La mélasse sous forme séchée permet d'éviter une détérioration des capsules. Ainsi, les micro-organismes sont préservés d'une altération éventuelle, y compris en présence de supports de formulation complémentaires (par exemple du lait en poudre).
- Dans le produit prébiotique et la composition symbiotique selon l'invention, les substances actives sont parfaitement homogénéisées.

Le produit prébiotique selon l'invention est à la fois une source d'énergie pour les micro-organismes et une source d'énergie directe pour l'hôte car il contient simultanément des sucres en particulier à faible index glycémique, des vitamines, des minéraux, des oligo-éléments et des acides aminés essentiels et non-essentiels, dont l'absorption est avantageusement facilitée par la combinaison avec les micro-organismes probiotiques. Vitamines, minéraux, oligo-éléments et acides aminés essentiels et non-essentiels sont des substances biologiquement actives, indispensables au fonctionnement physiologique de l'organisme sain ou pathologique, et qui favorisent l'état général de santé ou la santé globale de l'homme et de l'animal. Le produit prébiotique et la composition symbiotique de la présente invention représentent, de par leur composition en sucres en particulier à faible index glycémique, vitamines, minéraux, oligo-éléments et acides aminés essentiels et non-essentiels, un régime alimentaire très favorable à la santé de l'hôte.

Le produit prébiotique selon l'invention et la composition symbiotique selon l'invention sont utiles pour les indications non-thérapeutiques et thérapeutiques suivantes, qu'elles soient préventives, curatives ou palliatives, en général :
- ce qui concerne la sphère digestive: et plus particulièrement, amélioration de la constipation, diminution des processus inflammatoires de la partie inférieure du tractus intestinal, rééquilibrage bénéfique de la flore intestinale des personnes fragiles (enfants, personnes âgées, convalescents, malades) et de toute personne souffrant de carence.
- ce qui concerne les performances sportives: et plus particulièrement, stabilisation de la glycémie et augmentation de la glycogénolyse, augmentation des capacités de stockage des sucres par le muscle; amélioration de l'assimilation des nutriments; diminution de la fréquence des crampes (due à la concentration en magnésium et en potassium); amélioration de la récupération ; maintien de la masse musculaire.
- ce qui concerne la biologie des lipides: et plus particulièrement, diminution des concentrations du cholestérol total et LDL-cholestérol, augmentation du HDL-cholestérol et amélioration du rapport LDL/HDL, aide à lutter contre l'obésité et le risque athéromateux, amélioration des signes cliniques liés au syndrome métabolique.
- ce qui concerne l'immunité: et plus particulièrement, action stimulante de l'immunité avec diminution de la fréquence et de la sévérité des épisodes infectieux.
- ce qui concerne les fonctions cérébrales: et plus particulièrement, amélioration des fonctions mentales (jugement, mémoire, organisation mentale, orientation dans l'espace et le temps), et des fonctions perceptives (représentation du corps, représentation spatiale).
- ce qui concerne le bien-être: et plus particulièrement, diminution des sensations de fatigue, amélioration de l'humeur; diminution des effets de l'adrénaline produite par le stress.
- ce qui concerne les os et les cartilages: et plus particulièrement, amélioration de la consolidation de l'os après fracture, raideur des articulations, amélioration de l'arthrose et de l'ostéoporose.
- ce qui concerne le vieillissement: et plus particulièrement, action anti-âge par le maintien de l'équilibre acido-basique (due à la concentration en potassium); action anti-radicalaire (due à la concentration en manganèse), prévention de la perte musculaire liée à l'âge.

### DESCRIPTION DE MODES DE REALISATION DE L'INVENTION

Le produit prébiotique selon l'invention comprend de la mélasse séchée et une composition de polyosides. La composition symbiotique selon l'invention comprend le produit prébiotique ci-dessus, en association avec des micro-organismes (de préférence vivants, déshydratés et encapsulés).

Par « produit prébiotique » on entend un produit alimentaire, en partie non digestible par l'homme ou l'animal, susceptible de stimuler la croissance et l'activité des micro-organismes de la flore intestinale ou des micro-organismes probiotiques. Le produit prébiotique au sens de la présente demande est dépourvu de micro-organismes.

Par « micro-organismes probiotiques » on entend des micro-organismes susceptibles d'avoir une action bénéfique sur la santé de l'hôte humain ou animal auquel ils sont administrés.

L'invention est maintenant décrite plus en détail et de façon non limitative dans la description qui suit. Sauf mention contraire, les proportions sont données en valeurs massiques.

### Mélasse

Par « mélasse » on entend le co-produit constitué par le résidu sirupeux insusceptible de cristallisation qui est recueilli lors de la fabrication de produits sucrés (de préférence de sucres simples) à partir de végétaux.

Les végétaux en question peuvent être de préférence de la canne (dans le cadre de la production de saccharose) ou des betteraves mais également, éventuellement des céréales (dans le cadre de la production de glucose) ou des fruits (dans le cadre de la production de pulpe concentrée).

Typiquement, lors de la fabrication de sucre, on extrait un jus qui est purifié par la chaux. Le jus épuré est concentré sous pression, puis sous vide et cristallisé. Au fur et à mesure que les cristaux de sucre se forment, les résidus se concentrent dans la fraction liquide. La cristallisation peut par exemple être accomplie en trois jets, les cristaux de sucre étant à chaque fois enlevés avant de concentrer à nouveau le jus. Après la dernière cristallisation, le jus résiduel ne cristallise plus, même si on le concentre à nouveau. C'est ce jus qui est connu dans le domaine sous le nom de mélasse (ou mélasse brute).

Cette mélasse contient généralement de 65 à 80% de matières sèches, de préférence de 70 à 76%; elle contient généralement de 10 à 20% (en matière sèche) de matières minérales, de préférence de 12 à 15%; elle contient généralement de 2 à 20% (en matière sèche) de matières azotées, de préférence de 5 à 16%; et elle contient généralement de 50 à 75% (en matière sèche) de sucres, de préférence de 60 à 70%.

Plus précisément, la mélasse comprend généralement les composés suivants (les gammes de concentration étant fournies indépendamment les unes des autres) :
- de 30 à 66%, de préférence de 35 à 40% (en matière sèche) de saccharose ;
- de 1 à 10%, de préférence de 2,5 à 5% (en matière sèche) de glucose ;
- de 1 à 5%, de préférence de 2,5 à 5% (en matière sèche) de fructose ;
- de 0,2 à 2%, de préférence de 0,5 à 1 % (en matière sèche) de rhamnose ;
- de 0,2 à 1 %, de préférence de 0,4 à 0,7% (en matière sèche) de cuivre ;
- de 0,2 à 1 %, de préférence de 0,3 à 0,9% (en matière sèche) de calcium ;
- de 1 à 2%, de préférence de 1,4 à 1,7% (en matière sèche) de fer ;
- de 0,2 à 0,7%, de préférence de 0,3 à 0,6% (en matière sèche) de manganèse ;
- de 3,5 à 8,5%, de préférence de 4 à 5% (en matière sèche) de potassium ;
- de 0,2 à 1%, de préférence de 0,2 à 0,5% (en matière sèche) de magnésium ;
- de 0,2 à 1 %, de préférence de 0,2 à 0,5% (en matière sèche) de soufre ;
- de 0,1 à 0,5%, de préférence de 0,1 à 0,2% (en matière sèche) de pyridoxine (vitamine B6) ;
- de 7 à 8%, de préférence de 7 à 7,5% (en matière sèche) de choline ;
- de 0,1 à 1 %, de préférence de 0,5 à 0,7% (en matière sèche) de niacine (vitamine B3) ;
- de 0,1 à 0 ,3%, de préférence de 0,1 à 0,2% (en matière sèche) de riboflavine (vitamine B2) ;
- de 0 à 1 %, de préférence de 0,7 à 0,9% (en matière sèche) de thiamine (vitamine B1) ;
- de 1 à 5%, de préférence de 1,3 à 4% (en matière sèche) d'acide aspartique ;
- de 0,1 à 1 %, de préférence de 0,3 à 0,5% (en matière sèche) d'arginine ;
- de 2 à 5%, de préférence de 2,2 à 4,5% (en matière sèche) d'acide glutamique ;
- de 0,1 à 0,5%, de préférence de 0,11 à 0,21 % (en matière sèche) d'histidine.

La composition exacte de la mélasse dépend toutefois du procédé de production ainsi que de la matière première végétale.

Les mélasses de cannes à sucre et de betteraves sont connues en tant qu'appétant et liant pour les constituants de l'alimentation animale; ou en tant que milieu de culture pour les micro-organismes (essentiellement les levures de boulangerie); ou en tant qu'édulcorant dans l'alimentation humaine en particulier la confiserie.

Dans le cadre de l'invention, le terme de « mélasse séchée » désigne un produit contenant les mêmes ingrédients que la mélasse conventionnelle décrite ci-dessus, mais avec une teneur en eau inférieure à la teneur en eau de cette mélasse conventionnelle. Ainsi, la mélasse séchée présente de préférence une teneur en matières sèches supérieure ou égale à 80%, plus particulièrement supérieure ou égale à 85%, supérieure ou égale à 90% voire idéalement supérieure ou égale à 94%, 95% ou 96%.

En d'autres termes, la mélasse séchée utilisée dans le cadre de l'invention peut être qualifiée de « mélasse séchée à l'état brut », dans la mesure où la seule différence de composition entre la mélasse séchée selon l'invention et la mélasse conventionnelle réside dans la teneur en eau, ladite mélasse séchée n'ayant subi aucune transformation chimique, ni aucun ajout ou retrait d'ingrédient par rapport à la mélasse conventionnelle.

La mélasse séchée utilisée dans la présente invention est de préférence sous forme de poudre.

La mélasse séchée utilisée dans la présente invention est de préférence conforme à la réglementation française et / ou à la réglementation européenne relatives à l'alimentation. Ceci est effectué en prenant les mesures nécessaires lors de la culture des végétaux à l'origine de la mélasse, et lors de leur transport et traitement industriel. Notamment, la mélasse séchée est substantiellement exempte de pesticides et autres résidus au sens du règlement communautaire n° 178/2006 ensemble le règlement communautaire n° 396/2005.

### Fibres

La composition de polyosides utilisée dans le cadre de la présente invention est une composition comprenant des fibres solubles dans l'eau, de grade alimentaire. Ces fibres remplissent une fonction d'agent de charge et participent également à la fonction prébiotique.

Les polyosides sont des polymères constitués de plusieurs oses liés entre eux par des liaisons O-osidiques. Les oses peuvent être des trioses, tétroses, pentoses, hexoses ou heptoses, de préférence des hexoses. Parmi les hexoses, le glucose est préféré.

Les polyosides préférés dans le cadre de l'invention sont les polymères de glucose, et notamment des produits dérivés de l'amidon (par exemple hydrolysats), tels que la dextrine ou la maltodextrine, et les mélanges de ceux-ci.

De préférence, la composition de polyosides est une composition de dextrine.

La composition de polyosides selon l'invention présente une teneur en fibres supérieure ou égale à 80%, et par exemple d'environ 85% (en masse).

La teneur en fibres peut être déterminée au moyen de la méthode AOAC 2001-03 (méthode de chromatographie en phase liquide à haute performance, enzymatique et gravimétrique).

Cette teneur en fibres (polysaccharides à chaînes longues) est particulièrement utile pour masquer les sucres de petite taille de la mélasse lors du procédé de séchage (notamment par atomisation) et ainsi éviter la prise en masse.

La composition de polyosides peut être dérivée d'amidon végétal tel que l'amidon de blé ou de maïs, de préférence de maïs (afin notamment d'éviter la présence de gluten et autres substances allergènes). Elle peut être obtenue par un procédé de dextrinisation suivi d'un fractionnement (par exemple par chromatographie).

De préférence, la composition de polyosides comprend une teneur en matières sèches supérieure ou égale à 94%, 95% ou 96%.

La composition de polyosides présente de préférence une teneur en monosaccharides et en disaccharides inférieure ou égale à 5%, ou inférieure ou égale à 2%, ou inférieure ou égale à 1 %, ou inférieure ou égale à 0,5%. Cette teneur peut être déterminée par chromatographie en phase liquide à haute performance.

La composition de polyosides présente de préférence une masse moléculaire moyenne en poids de 4000 à 7000 daltons, ou de 4500 à 6000 daltons ou 4800 à 5200 daltons (par exemple d'environ 5000 daltons). Elle présente de préférence une masse moléculaire moyenne en nombre de 1500 à 4500 daltons, ou de 2000 à 3500 daltons ou 2400 à 2800 daltons (par exemple d'environ 2600 daltons). Cette masse moléculaire peut être déterminée par chromatographie d'exclusion de taille à haute performance.

La composition de polyosides présente de préférence une viscosité, à 60% dans de l'eau à 20°C, de 1000 à 2500 mPa.s, ou de 1250 à 2000 mPa.s ou de 1400 à 1600 mPa.s (par exemple d'environ 1500 mPa.s). Elle présente de préférence une viscosité, à 50% dans de l'eau à 20°C, de 100 à 300 mPa.s, ou de 150 à 250 mPa.s ou de 180 à 220 mPa.s (par exemple d'environ 200 mPa.s).

Les propriétés de masse moléculaire et de viscosité ci-dessus facilitent la fabrication du produit prébiotique sous une forme homogène.

La composition de polyosides, lorsqu'il s'agit d'une composition de dextrine, comme cela est préféré, présente de préférence le profil de liaisons glycosidiques suivant : de 45 à 55% de liaisons (1,4), de 25 à 35% de liaisons (1,6), de 5 à 15% de liaisons (1,2) et de 5 à 15% de liaisons (1,3) ; et par exemple : environ 50% de liaisons (1,4), environ 30% de liaisons (1,6), environ 10% de liaisons (1,2) et environ 10% de liaisons (1,3). Ce profil de liaisons glycosidiques peut être déterminé par spectrométrie de masse après méthylation. La proportion relativement élevée de liaisons (1,2) et (1,3) (essentiellement non digestibles) assure une digestibilité relativement fiable des polyosides, ce qui est bénéfique pour la fonction prébiotique.

Le produit commercialisé par Roquette sous la référence Nutriose® 06 FM présente l'ensemble des propriétés ci-dessus et constitue donc un ingrédient particulièrement approprié pour la fabrication du produit prébiotique selon l'invention.

### Micro-organismes

Les micro-organismes utilisés dans le cadre de l'invention peuvent être tous micro-organismes et en particulier tous micro-organismes connus pour leur action bénéfique sur la santé humaine ou animale (micro-organismes probiotiques). Il peut s'agir de bactéries ou de levures.

Parmi les genres de micro-organismes préférés, on peut notamment citer les genres *Bifidobacterium, Lactobacillus, Lactococcus, Streptococcus, Pediococcus, Enterococcus, Propionibacterium, Saccharomyces* et *Kluyveromyces.*

Des exemples d'espèces de micro-organismes préférés sont *Lactobacillus acidophilus, Lactobacillus plantarum, Lactobacillus casei, Lactobacillus rhamnosus, Lactobacillus zeae, Lactobacillus salivarius, Lactobacillus lactis, Lactobacillus helveticus, Lactobacillus reuteri, Lactobacillus amylovorus, Lactobacillus crispatus, Lactobacillus curvatus, Lactobacillus delbrueckii* (notamment *Lactobacillus delbrueckii ssp. delbrueckii* et *Lactobacillus delbrueckii ssp. bulgaricus*), *Lactobacillus gasseri, Lactobacillus johnsonii, Lactobacillus fermentum, Lactobacillus brevis, Streptococcus thermophilus, Streptococcus* salivarius, *Lactococcus lactis* (notamment *Lactococcus lactis ssp. lactis* et *Lactococcus lactis ssp. cremoris*), *Leuconostoc sp., Enterococcus faecium, Pediococcus pentosaceus, Pediococcus acidilactici, Bifidobacterium longum, Bifidobacterium breve, Bifidobacterium bifidum, Bifidobacterium infantis, Bifidobacterium lactis, Bifidobacterium helveticus, Saccharomyces cerevisiae, Saccharomyces boulardii* ainsi que les mélanges de ceux-ci.

L'espèce *Bifidobacterium longum* est particulièrement préférée.

Les micro-organismes présents dans la composition symbiotique selon l'invention sont de préférence vivants, déshydratés et encapsulés. Le terme « vivants » signifie qu'au moins une partie des micro-organismes sont vivants (c'est-à-dire susceptibles de croître dans un milieu de culture approprié), étant entendu qu'une certaine fraction des micro-organismes est nécessairement présente sous forme inactive.

De préférence, la composition contient des micro-organismes en une quantité d'au moins 10⁵, plus particulièrement au moins 10⁷, idéalement au moins 5×10⁷ cfu/g de composition.

La forme encapsulée garantit une meilleure préservation des micro-organismes, en les protégeant de l'environnement et en présentant à la fois une bonne stabilité chimique et une bonne résistance aux contraintes mécaniques.

De préférence, ces micro-organismes vivants sont sous forme d'agglomérats, déshydratés et enrobés d'une couche hydrophobe. La couche hydrophobe peut être préférentiellement une couche d'acide gras, de cire ou d'un mélange de ceux-ci. Les particules enrobées présentent de préférence un diamètre moyen compris entre 100 µm et 5 mm, de manière plus particulièrement préférée entre 300 µm et 2 mm.

Il est renvoyé au document WO 01/68808 qui décrit en détail le procédé d'obtention de tels micro-organismes vivants, déshydratés et encapsulés. Ces produits sont également disponibles dans le commerce, notamment par Lallemand (institut Rosell) sous la marque Probiocap™.

### Produit prébiotique et composition symbiotique

Le produit prébiotique selon l'invention, tout comme la composition symbiotique sont de préférence sous forme pulvérulente. Leur teneur (massique) en eau est de préférence inférieure ou égale à 6%, plus particulièrement inférieure ou égale à 5%, de manière plus particulièrement préférée inférieure ou égale à 4%.

Le produit prébiotique peut comprendre par exemple, en matière sèche : de 12 à 25% de saccharose, de préférence de 15 à 22% de saccharose ; de 3 à 6% de fructose, de préférence de 3,5 à 5% de fructose ; de 1 à 3% de glucose, de préférence de 1,3 à 2% de glucose ; de 1,5 à 4% de potassium, de préférence de 2,1 à 2,7% de potassium ; de 1,5 à 4% de magnésium, de préférence de 2,1 à 2,7% de magnésium ; de 0,01 % à 0,2% de vitamine B6, de préférence de 0,02 à 0,08% de vitamine B6.

Le produit prébiotique tout comme la composition symbiotique selon l'invention peuvent également contenir divers additifs tels que un ou plusieurs arômes, excipients, colorants et agents de formulation ainsi que toute substance d'origine naturelle organique, minérale et végétale autre que sucres, vitamines, minéraux, oligoéléments et acides aminés, en une concentration massique de préférence comprise entre 0,01 à 50%, plus particulièrement entre 0,1 et 10%.

### Procédé

Le produit prébiotique selon l'invention peut être fabriqué par mélange de mélasse avec la composition de polyosides ci-dessus, pour fournir un pré-mélange, puis séchage du pré-mélange.

La composition symbiotique selon l'invention peut être fabriquée soit directement par addition de micro-organismes au produit prébiotique selon l'invention (donc, après séchage), et mélange ; soit par addition de micro-organismes au stade du pré-mélange, avant séchage.

La mélasse et la composition de polyosides peuvent être de préférence mélangées dans les rapports suivants : de 20 à 60% de mélasse et de 40 à 80% de composition de polyosides, et notamment de 30 à 50% de mélasse et de 50 à 70% de composition de polyosides.

Tout type de mélangeur conventionnel peut être utilisé pour le mélange des fibres de polyoside et de la mélasse. Lors de la fabrication du pré-mélange, les fibres de polyoside sont imprégnées avec la mélasse. A ce stade, il est possible d'ajouter de l'eau afin de faciliter l'imprégnation, dans le cas où la mélasse n'en contient pas suffisamment. Pour une imprégnation optimale, on peut choisir d'ajuster le taux de matière sèche du pré-mélange entre 30 et 60%, de préférence entre 40 et 50% (en masse), par ajout d'eau.

L'eau peut éventuellement être ajoutée sous forme d'un mélange d'eau et d'additifs, ou bien sous forme de jus de fruits ou jus de légumes.

Les micro-organismes (de préférence vivants, déshydratés et encapsulés) peuvent être ajoutés et mélangés dans le même mélangeur, qu'ils soient incorporés au stade du pré-mélange (avant séchage) ou directement au produit prébiotique (après séchage). La quantité de micro-organismes ajoutés est de préférence comprise entre 10 et 100 mg (en matière sèche) par gramme de pré-mélange, plus particulièrement entre 20 et 60 mg, idéalement entre 30 et 50 mg.

Les étapes de pré-mélange ou mélange peuvent être effectuées à température ambiante.

Lorsque les micro-organismes sont ajoutés au stade du pré-mélange, il est souhaitable que l'étape de séchage intervienne aussi rapidement que possible après l'ajout des micro-organismes, afin d'éviter toute dégradation ou tout déclenchement d'activité de ceux-ci au contact d'un milieu contenant de l'eau et des sucres. De préférence, la durée séparant l'ajout des micro-organismes du séchage est inférieure à 1 h/kg de matière sèche, plus particulièrement inférieure à 30 minutes/kg de matière sèche, idéalement inférieure à 15 minutes/kg de matière sèche.

De préférence, la durée séparant l'ajout des micro-organismes du séchage est inférieure à 1 heure, plus particulièrement inférieure à 30 minutes, idéalement inférieure à 15 minutes, voire à 10 minutes ou 5 minutes.

Le séchage peut être effectué au moyen d'une étuve sous vide, par dessiccation, par lyophilisation, par rayonnement micro-ondes, au moyen d'un lit fluidisé ou par atomisation. L'atomisation est avantageuse pour conserver les propriétés nutritionnelles de la mélasse, car elle est effectuée à une température modérée (de l'ordre de 110°C à 180°C, idéalement entre 130°C et 160°C, et plus particulièrement entre 150 et 160°C, par exemple 155°C en entrée et de l'ordre de 70°C à 90°C, et plus particulièrement entre 80°C et 90°C, par exemple 85°C en sortie).

L'atomisation consiste à injecter le pré-mélange sous la forme d'un fin brouillard en haut d'une tour en même temps qu'un courant d'air chaud. Sous l'effet de la chaleur, l'eau s'évapore et charge l'air en eau. La composition séchée est récupérée en bas de la tour d'atomisation. Le pH du pré-mélange peut être ajusté à une valeur de 5 à 7,5 et idéalement de 7 à 7,5, afin de masquer les sites hydrophiles et d'améliorer l'atomisation.

Après séchage, on peut procéder à un tamisage.

Le produit prébiotique et la composition symbiotique selon l'invention sont de préférence sous forme de poudre et peuvent être conditionnés en sachets, flacons ou autres contenants, avec ou sans la présence de produit dessiccateur. Le produit prébiotique et la composition symbiotique selon l'invention peuvent être utilisés en tant qu'additifs pour tout produit alimentaire, alicament, complément alimentaire ou diététique, notamment: produit diététique sous forme de barre énergétique, de poudre à diluer, de gélules, de comprimés; produit déshydraté tel que chicorée, chocolat, café; plantes déshydratées telles que thé, tisanes; laitages déshydratés tels que yaourts, poudres et produits à base de soja; protéines déshydratées; biscuits; laitages et produits frais à base de soja; compotes; crèmes desserts; crèmes glacées; produit d'alimentation animale sous toutes ses formes (blocs, granulés, comprimés, poudres...).

Le produit prébiotique et la composition symbiotique selon l'invention peuvent être commercialisés tels quels et ajoutés directement par le consommateur à un produit alimentaire. Alternativement, ils peuvent être incorporés en tant qu'additifs ou ingrédients à un produit alimentaire au stade de sa production.

Le produit prébiotique et la composition symbiotique selon l'invention peuvent également être utilisés pour la fabrication d'un médicament, soit directement sous forme de poudre à disperser dans de l'eau (le produit prébiotique ou la composition symbiotique étant alors en combinaison ou non avec un ou plusieurs additifs et/ou excipients pharmaceutiquement acceptables), soit sous une quelconque forme galénique connue de l'homme du métier: comme par exemple formulations pour les voies d'administration locale telles que pommade, crème, lotion, gel, émulsion, shampoing, cire, masque, mousse à raser, crème dépilatoire, capsule ou gélule vaginale, patchs, implants, suppositoires; de préférence une forme galénique pour voie orale, notamment gélules, capsules, comprimés, tablettes à croquer ou granules, en combinaison avec un ou plusieurs véhicules, additifs, ingrédients et/ou excipients pharmaceutiquement acceptables.

Le produit prébiotique et la composition symbiotique selon l'invention peuvent également être utilisés pour la fabrication d'un produit cosmétique, pour application sur les parties superficielles du corps humain (épiderme, systèmes pileux et capillaires, ongles, lèvres, organes génitaux externes, dents, muqueuses buccales) en vue d'en modifier l'aspect, de les protéger, de les nettoyer, de les maintenir en bon état, de les parfumer et / ou de corriger les odeurs corporelles. Le produit cosmétique peut notamment être sous forme de crème, émulsion, lotion, gel, huile, masque de beauté, fond de teint, poudre, savon, parfum, shampooing, produit déodorant, produit antisudoral, sel, mousse ou autre.

La composition symbiotique selon l'invention présente des propriétés de stabilité satisfaisantes, voire améliorées par rapport aux propriétés de stabilité des micro-organismes vivants, déshydratés et encapsulés pris isolément, et ce sur une durée d'au moins 1 mois, de préférence d'au moins 6 mois, de manière plus particulièrement préférée d'au moins 1 an, idéalement d'au moins 2 ans voire d'au moins 3 ans. La stabilité est évaluée en mesurant le nombre de micro-organismes viables dans la composition à différentes dates successives après la fabrication.

### Utilisation

Le produit prébiotique, la composition symbiotique, ainsi que les produits alimentaires/diététiques et cosmétiques ou les médicaments qui en sont dérivés, sont indiqués pour un usage humain ou animal.

Les composés actifs de la composition, à savoir la mélasse, les polyosides et le cas échéant les micro-organismes, ont des effets qui s'additionnent, voire agissent en synergie, afin de :
- prévenir un état de maladie ; et/ou
- participer à la perte de poids et/ou faciliter la perte de poids et/ou traiter ou prévenir l'obésité et/ou traiter ou soulager les désordres ou maladies liées à ou causées par l'obésité ou le surpoids ; et/ou
- renforcer l'organisme face au stress, à la fatigue, à un état de carence, à des troubles de l'humeur ; et/ou
- contribuer à préserver l'homéostasie comme aider l'organisme à la régulation de la tension artérielle ; et/ou
- faciliter une convalescence ; et/ou
- stimuler les défenses immunitaires ; et/ou
- stimuler les fonctions digestives et améliorer le confort digestif ; et/ou
- assurer une meilleure biodisponibilité des nutriments et améliorer l'assimilation de nutriments essentiels ; et/ou
- apporter de l'énergie à absorption lente à l'organisme ; et/ou
- prévenir la fonte musculaire liée à l'âge, consécutif à un affaiblissement de l'organisme ou à un état de maladie ; et / ou
- protéger contre les infections de la sphère ORL et de la sphère gynécologique (mycoses vaginales...).

Les sujets jeunes, les sujets adultes, les sujets âgés, les sujets malades, les sujets convalescents, les sujets carencés, les sujets sportifs, constituent des populations pour lesquelles la composition selon l'invention est particulièrement utile.

Par extension, tout sujet voulant prendre soin de sa santé et prévenir un état de maladie trouvera la présente invention opportune pour améliorer et maintenir son état de bien-être physique et mental.

En outre, la teneur en cuivre du produit prébiotique ou de la composition symbiotique selon l'invention, de préférence supérieure à 0,4% en masse, permet de conférer au produit prébiotique ou à la composition symbiotique un effet anti-anémiant, antioxydant, anti-inflammatoire et anti-infectieux.

La teneur en fer du produit prébiotique ou de la composition symbiotique selon l'invention, de préférence supérieure à 1,4% en masse, permet de conférer au produit prébiotique ou à la composition symbiotique un effet anti-anémiant.

La teneur en calcium du produit prébiotique ou de la composition symbiotique selon l'invention, de préférence supérieure à 0,3% en masse, permet de conférer au produit prébiotique ou à la composition symbiotique un effet de maintien de la résistance et de la densité des os, de la conduction nerveuse, de la contraction musculaire, ainsi qu'un effet de régulation de l'activité enzymatique et des membranes cellulaires.

La teneur en potassium du produit prébiotique ou de la composition symbiotique selon l'invention, de préférence supérieure à 4% en masse, permet de conférer au produit prébiotique ou à la composition symbiotique un effet de régulation de la conduction nerveuse normale ou pathologique, de l'activité musculaire et de l'équilibre acido-basique, et de prévention ou traitement, de l'athérosclérose, de l'hypertension, des maladies inflammatoires chroniques intestinales.

La teneur en manganèse du produit prébiotique ou de la composition symbiotique selon l'invention, de préférence supérieure à 0,3% en masse, permet de conférer au produit prébiotique ou à la composition symbiotique un effet d'activation enzymatique de protection des cellules contre les radicaux libres.

La teneur en magnésium du produit prébiotique ou de la composition symbiotique selon l'invention, de préférence supérieure à 0,2% en masse, permet de réguler l'activité neuronale et musculaire.

La teneur en fructose du produit prébiotique ou de la composition symbiotique selon l'invention, de préférence supérieure à 2,5% en masse, permet de conférer au produit prébiotique ou à la composition symbiotique un effet hypoglycémiant et de stabilisation de la glycémie, d'amélioration de l'endurance.

La présence de choline dans le produit prébiotique ou la composition symbiotique selon l'invention permet d'améliorer les capacités de récupération de l'organisme, prévient la fatigue musculaire, augmente les performances chez le sportif, freine la diminution des performances physiques et cognitives liées à l'âge.

### EXEMPLES

Les exemples suivants illustrent l'invention sans la limiter.

### Exemple 1 - fabrication de composition symbiotique

Une composition symbiotique selon l'invention est préparée à partir des composés suivants :
- mélasse de canne à sucre à l'état brut d'origine Guadeloupe, sucrerie GARDEL, de type alimentaire spécialement prélevée pour la préparation ;
- micro-organismes vivants, déshydratés et encapsulés: bactéries de type *Bifidobacterium longum,* commercialisées par Lallemand (France) sous la référence Probiocap Rosell-175 ME; la caractérisation microbiologique de ce composé, fournie par la société Lallemand, est la suivante: *Bifidobacterium longum >* 5×10¹⁰ cfu/g; staphylocoques < 10 cfu/g; levures et moisissures < 10³ cfu/g; saprophytes < 10⁴ cfu/g; *Pseudomonas <* 25 cfu/g; bacilles < 10⁴ cfu/g; salmonelles < 1 cfu par 25 g; coliformes < 1 cfu/g.
- composition de polyosides : fibres de dextrine Nutriose® 06 FM (Roquette, France).

On effectue une caractérisation microbiologique de la mélasse avant son utilisation. A cette fin, la mélasse est diluée dans un bouillon LT100, au 1/10ème, puis ensemencée :
- pour le dénombrement des germes totaux aérobies, sur boîte de Petri dans une gélose trypticase soja (peptone de caséine: 17,0 g/l - peptone de farine de soja: 3,0 g/l - D(+)- glucose: 2,5 g/l - chlorure de sodium: 5,0 g/l - phosphate dipotassique: 2,5 g/l - eau: 1000 ml, pH: 7,3 ± 0,2) ;
- pour le dénombrement des levures et des moisissures, sur un milieu Sabouraud Glucosée Chloramphénicol - gélose (SAB, Biomérieux, France).

Dans les deux cas, les milieux gélosés sont placés dans une étuve pendant 5 jours, à 37°C. Puis, les colonies sont comptées et les résultats sont exprimés en cfu/ml.

Les germes totaux ont une concentration inférieure à 1000 cfu/g et les levures et moisissures, une concentration inférieure à 100 cfu/g.

Préparation du pré-mélange: on mélange 40% de mélasse à 60% de composition de polyosides dans un triblender inox (F2116MD-MBV-F, Tri-clover USA) préalablement désinfecté. On effectue une dilution à 10% avec de l'eau. Après homogénéisation, on mesure la teneur en matière sèche du pré-mélange. A cette fin, une fraction du pré-mélange est séchée à l'étuve (Memmert GmbH, Allemagne) à 105°C, pendant 2 heures. Le résidu sec est pesé avec une balance Sartorius 182 g à une précision de 10⁻⁴ g. On vérifie que la teneur (massique) en matière sèche du pré-mélange est comprise entre 40% et 50%.

On ajoute 40 mg/g (en matières sèches) de probiotiques au pré-mélange, et on mélange le tout avec le triblender à la vitesse de 100 trs/min, à une température n'excédant pas 25°C pour obtenir le mélange.

Obtention de la composition finale: la composition finale est obtenue par atomisation du mélange. La tour de séchage par atomisation comprend un chauffage par résistance électrique, et une pulvérisation avec turbine ou buse bi-fluide. Le temps de séjour dans la chambre est de 15 à 24 s, le débit d'air est d'environ 500 kg/h. La température d'entrée est de 155°C, la température de sortie est de 85°C. La différence de pression du cyclone est de 200 mm Hg.

Traitement final: la composition finale est tamisée sur un tamis vibrant inox (Vibrowest, Italie) de 250 µm, à 1000-1450 trs/min, 0,5-1,85 kW, 30 s/kg. La composition est ensuite immédiatement conditionnée en sachets pour éviter toute prise d'humidité et toute contamination.

La composition finale obtenue se présente sous la forme d'une fine poudre brune présentant l'odeur caractéristique de la mélasse. La perte à la dessiccation est inférieure à 6%. La solubilité de la poudre obtenue est de 10%.

Quinze jours après la fabrication de la composition, la quantité de probiotiques de type *Bifidobacterium longum* viables dans la composition est mesurée par la société Lallemand. Sur trois échantillons distincts, la population de *Bifidobacterium longum* est évaluée respectivement à 4,2×10⁷ cfu/g, 5,3×10⁷ cfu/g et 2,35×10⁷ cfu/g.

### Exemple 2 - effet de la composition symbiotique sur la viabilité cellulaire

La composition obtenue à l'exemple 1 est évaluée du point de vue de la viabilité des cellules issues d'un adénocarcinome colorectal humain, par la méthode de réduction du MTT (bromure de méthylthiazolyldiphényl-tetrazolium). Les cellules (lignée Caco-2, clone TC-7), sont cultivées en monocouche dans des nacelles jusqu'à confluence dans le milieu DMEM/MEM Acane additionné de pénicilline (Sigma, 50 UI/mL), de stroptomycine (Sigma, 50 mg/mL), de gentamycine (Sigma, 10 mg/mL) et de sérum de veau foetal (Sigma, 10%, v/v).

La composition finale est diluée à 1 µg/mL, 10 µg/mL, 100 µg/mL, 1 mg/mL, 10 mg/mL et 100 mg/mL et mise en présence des cellules cultivées en nacelle pendant 48h, à 37°C en atmosphère humide contenant 5% de CO₂. L'EGF *(Epithelial Growth Factor)* est utilisé comme produit de référence dans le test de la prolifération des cellules TC-7.

La viabilité cellulaire est mesurée selon la méthode de réduction du MTT. La réduction du MTT en bleu de formazan est évaluée par une mesure de la densité optique, proportionnelle au nombre de cellules métaboliquement actives, déterminée par spectrophotométrie à 540 nm.

Les résultats sont exprimés en unités arbitraires de densité optique (D.O) par échantillon et en pourcentage de variation par rapport au groupe témoin. Les tests statistiques sont effectués sur les valeurs de D.O. La différence entre les moyennes des différents groupes 'témoin' *versus* groupe 'traité' est déterminée par analyse de la variance à un facteur (Anova 1, σ <0,05) et la moyenne des groupes traités à celle du groupe témoin est déterminée par le test de Dunnett (σ <0,1).

Les résultats sont représentés dans le tableau 1 ci-dessous.

**Tableau 1: effet de la composition de l'exemple 2 sur la viabilité des cellules TC-7 après 48 heures d'incubation**

| | Concentration de la composition, en mg/mL | | | | | | |
|---|---|---|---|---|---|---|---|
| | Témoin | 0,001 | 0,01 | 0,1 | 1 | 10 | 100 |
| DO | 1,115 | 1,263 | 1,081 | 1,343 | 1,405 | 1,354 | 0,766 |
| | 1,259 | 1,323 | 1,162 | 1,36 | 1,311 | 1,402 | 0,766 |
| | 1,181 | 1,259 | 1,243 | 1,323 | 1,402 | 1,339 | 0,726 |
| Moyenne | 1,185 | 1,282 | 1,162 | 1,342*** | 1,373*** | 1,365*** | 0,753*** |
| Ecart type | 0,072 | 0,036 | 0,081 | 0,019 | 0,053 | 0,033 | 0,023 |
| Viabilité cellulaire en pourcentage du "témoin" | 100 | 108 | 98 | 113 | 116 | 115 | 64 |

| | | | | | | | |
|---|---|---|---|---|---|---|---|
| *** = moyenne significativement différente de celle du groupe témoin (p<0,01). | | | | | | | |

On constate que la composition finale à des concentrations inférieures ou égales à 10 mg/mL ne modifie pas la viabilité des cellules TC-7, après 48 heures d'incubation et ne présente donc aucun effet cytotoxique.

### Exemple 3 - stabilité à 3 mois

La composition selon l'invention est évaluée du point de vue de la stabilité à 3 mois après conservation à température ambiante :
- pour la composition de l'exemple 1 ;
- pour une composition similaire à celle de l'exemple 1 obtenue selon un protocole modifié (mélange du probiotique au prébiotique après séchage) ;
- la composition étant conservée soit seule, soit en mélange avec un support de poudre de lait (de marque Régilait®).

Pour le mélange avec la poudre de lait, on mélange 20 g de support avec 4 g de composition symbiotique, dans des flacons de verre stérile, placés à l'abri de la lumière et à température ambiante. On effectue un dénombrement à la date initiale, puis à 1 mois et à 3 mois, sur le mélange avec la poudre de lait et sur la composition symbiotique conservée seule.

Le dénombrement est effectué de la façon suivante :
- 1 g de produit seul ou en mélange a été mis en suspension dans 9 ml de tryptone-sel (10⁻¹) ;
- agitation douce pendant 1 minute afin de permettre la dispersion ;
- réalisation de dilutions sériées au 1/10 à l'aide de tryptone-sel jusqu'à la dilution 10⁻⁶;
- réalisation d'un ensemencement en profondeur de 2 × 1 mL de chacune des dilutions réalisées à l'aide de gélose MRS ;
- incubation sous anaérobiose, à 37 ± 2 °C pendant 48 heures.

Le dénombrement des micro-organismes pour la composition sans poudre de lait est ramené à 20% pour pouvoir être comparée avec celui du mélange avec la poudre de lait.

On observe, que, après 3 mois de stockage, la population de la composition de l'exemple 1 présente une réduction de l'ordre d'un facteur 100, alors que la population de la composition de l'exemple 1 en mélange avec la poudre de lait est approximativement maintenue ; après 3 mois de stockage, la population de la composition de l'exemple 1 modifié présente une réduction de l'ordre d'un facteur 10, alors que la population de la composition de l'exemple 1 modifié en mélange avec la poudre de lait est approximativement maintenue.

## Revendications

1. Produit prébiotique comprenant:
- de la mélasse séchée, dans laquelle la mélasse séchée présente une teneur en matières sèches supérieure ou égale à 80%, en mélange avec
- une composition de polyosides,
dans laquelle la composition de polyosides présente une teneur en fibres supérieure ou égale à 80%.

2. Produit prébiotique selon la revendication 1, dans lequel la composition de polyosides est une composition de dextrine ou de maltodextrine, de préférence une composition de dextrine dérivée de l'amidon de maïs.

3. Produit prébiotique selon la revendication 1 ou 2, comprenant une proportion massique totale d'eau inférieure ou égale à 6%, de préférence inférieure ou égale à 5%, de manière plus particulièrement préférée inférieure à 4%.

4. Produit prébiotique selon l'une des revendications 1 à 3, dans lequel la composition de polyosides présente :
- une teneur en monosaccharides et en disaccharides inférieure ou égale à 5%, de préférence inférieure ou égale à 2%, de manière plus particulièrement préférée inférieure ou égale à 1% et idéalement inférieure ou égale à 0,5% ; et / ou
- une masse moléculaire moyenne en poids de 4000 à 7000 daltons, de préférence de 4500 à 6000 daltons et de manière plus particulièrement préférée de 4800 à 5200 daltons ; et / ou
- une viscosité, à 60% dans de l'eau à 20°C, de 1000 à 2500 mPa.s, de préférence de 1250 à 2000 mPa.s et de manière plus particulièrement préférée de 1400 à 1600 mPa.s ; et / ou
- de 45 à 55% de liaisons glycosidiques (1,4), de 25 à 35% de liaisons glycosidiques (1,6), de 5 à 15% de liaisons glycosidiques (1,2) et de 5 à 15% de liaisons glycosidiques (1,3).

5. Produit prébiotique selon l'une des revendications 1 à 4, comprenant, en proportions massiques : de 50 à 80% de la composition de polyosides et de 20 à 50% de mélasse séchée, de préférence de 60 à 70% de la composition de polyosides et de 30 à 40% de mélasse séchée.

6. Produit prébiotique selon l'une des revendications 1 à 5, dans lequel la mélasse est choisie parmi les mélasses de canne, de betterave, de fruits, de céréales et leurs mélanges, et de préférence parmi les mélasses de canne, de betterave et leurs mélanges, et de manière plus particulièrement préférée est une mélasse de canne.

7. Composition symbiotique comprenant le produit prébiotique selon l'une des revendications 1 à 6, en mélange avec une composition de micro-organismes probiotiques, de préférence des micro-organismes vivants, déshydratés et encapsulés.

8. Composition symbiotique selon la revendication 7, comprenant en proportions massiques :
- de 10 à 50% de mélasse séchée, de préférence de 15 à 45%, de manière plus particulièrement préférée de 20 à 40% ;
- de 15 à 70% de la composition de polyosides, de préférence de 20 à 65%, de manière plus particulièrement préférée de 35 à 60% ; et
- de 4 à 50% de la composition de micro-organismes probiotiques, de préférence de 10 à 40%, de manière plus particulièrement préférée de 20 à 30%;
et / ou comprenant une quantité de micro-organismes probiotiques vivants d'au moins 10⁵ cfu/g, de préférence au moins 10⁷ cfu/g, idéalement au moins 5×10⁷ cfu/g.

9. Composition symbiotique selon la revendication 7 ou 8, dans laquelle les micro-organismes probiotiques sont choisis parmi les bactéries ou levures des genres *Bifidobacterium, Lactobacillus, Lactococcus, Streptococcus, Pediococcus, Enterococcus, Propionibacterium, Saccharomyces, Kluyveromyces* et leurs mélanges, et de préférence parmi les espèces *Bifidobacterium longum, Lactobacillus casei, Lactobacillus rhamnosus, Lactobacillus helveticus* et *Saccharomyces torula.*

10. Produit alimentaire ou diététique comprenant le produit prébiotique selon l'une des revendications 1 à 6, ou la composition symbiotique selon l'une des revendications 7 à 9.

11. Produit cosmétique comprenant le produit prébiotique selon l'une des revendications 1 à 6, ou la composition symbiotique selon l'une des revendications 7 à 9.

12. Médicament comprenant le produit prébiotique selon l'une des revendications 1 à 6, ou la composition symbiotique selon l'une des revendications 7 à 9, sous forme de comprimés, gélules, poudre ou granules.

13. Procédé de fabrication d'un produit prébiotique selon l'une des revendications 1 à 6, comprenant une étape de mélange de mélasse avec la composition de polyosides pour obtenir un pré-mélange, puis une étape de séchage du pré-mélange.

14. Procédé de fabrication d'une composition symbiotique selon l'une des revendications 7 à 9, comprenant :
- une étape de mélange de mélasse avec la composition de polyosides pour obtenir un pré-mélange, puis une étape de séchage du pré-mélange, puis une étape de mélange du pré-mélange séché avec la composition de micro-organismes probiotiques ; ou
- une étape de mélange de mélasse avec la composition de polyosides et la composition de micro-organismes probiotiques pour obtenir un pré-mélange, puis une étape de séchage du pré-mélange.

15. Procédé selon la revendication 13 ou 14, dans lequel l'étape de séchage est effectuée au moyen d'une étuve sous vide, par lyophilisation, par rayonnement micro-ondes, au moyen d'un lit fluidisé ou par atomisation, et de préférence par atomisation ; et / ou dans lequel le taux de matière sèche du pré-mélange avant séchage est diminué par ajout d'eau, de préférence jusqu'à une valeur de 30 à 60%, de manière plus particulièrement préférée de 40 à 50% en masse.

## Patentansprüche

1. Präbiotisches Produkt, das Folgendes umfasst:
- getrocknete Melasse, wobei die getrocknete Melasse einen Gehalt an Trockenmaterialien aufweist, der größer als oder gleich 80% ist, im Gemisch mit
- einer Polyosidzusammensetzung,
wobei die Polyosidzusammensetzung einen Gehalt an Fasern aufweist, der größer als oder gleich 80% ist.

2. Präbiotisches Produkt nach Anspruch 1, wobei die Polyosidzusammensetzung eine Dextrin- oder Maltodextrinzusammensetzung ist, vorzugsweise eine Dextrinzusammensetzung, die aus Maisstärke abgeleitet ist.

3. Präbiotisches Produkt nach Anspruch 1 oder 2, das einen Gesamtmassenanteil von Wasser umfasst, der kleiner als oder gleich 6% ist, vorzugsweise kleiner als oder gleich 5%, besonders bevorzugt kleiner als 4%.

4. Präbiotisches Produkt nach einem der Ansprüche 1 bis 3, wobei die Polyosidzusammensetzung Folgendes aufweist:
- einen Gehalt an Monosacchariden und an Disacchariden, der kleiner als oder gleich 5% ist, vorzugsweise kleiner als oder gleich 2%, besonders bevorzugt kleiner als oder gleich 1% und idealerweise kleiner als oder gleich 0,5%; und/oder
- ein mittleres Molekulargewicht von 4000 bis 7000 Dalton, vorzugsweise 4500 bis 6000 Dalton und besonders bevorzugt 4800 bis 5200 Dalton; und/oder
- eine Viskosität bei 60% in Wasser von 20 °C von 1000 bis 2500 mPa.s, vorzugsweise 1250 bis 2000 mPa.s und besonders bevorzugt 1400 bis 1600 mPa.s; und/oder
- 45 bis 55% glykosidische (1,4) Bindungen, 25 bis 35% glykosidische (1,6) Bindungen, 5 bis 15% glykosidische (1,2) Bindungen und 5 bis 15% glykosidische (1,3) Bindungen.

5. Präbiotisches Produkt nach einem der Ansprüche 1 bis 4, das in Massenanteilen Folgendes umfasst: 50 bis 80% der Polyosidzusammensetzung und 20 bis 50% getrocknete Melasse, vorzugsweise 60 bis 70% der Polyosidzusammensetzung und 30 bis 40% getrocknete Melasse.

6. Präbiotisches Produkt nach einem der Ansprüche 1 bis 5, wobei die Melasse ausgewählt ist aus Melassen von Zuckerrohr, Zuckerrübe, Früchten, Getreiden und deren Gemischen und vorzugsweise aus Melassen von Zuckerrohr, Zuckerrübe und deren Gemischen und wobei die Melasse besonders bevorzugt eine Zuckerrohrmelasse ist.

7. Symbiotische Zusammensetzung, die ein präbiotisches Produkt nach einem der Ansprüche 1 bis 6 umfasst, im Gemisch mit einer Zusammensetzung von probiotischen Mikroorganismen, vorzugsweise lebende, dehydrierte und eingekapselte Mikroorganismen.

8. Symbiotische Zusammensetzung nach Anspruch 7, die in Massenanteilen Folgendes umfasst:
- 10 bis 50% getrocknete Melasse, vorzugsweise 15 bis 45%, besonders bevorzugt 20 bis 40%;
- 15 bis 70% der Polyosidzusammensetzung, vorzugsweise 20 bis 65%, insbesondere bevorzugt 35 bis 60%; und
- 4 bis 50% der Zusammensetzung von probiotischen Mikroorganismen, vorzugsweise 10 bis 40%, besonders bevorzugt 20 bis 30%;
und/oder eine Menge von lebenden probiotischen Mikroorganismen von mindestens 10⁵ cfu/g umfasst, vorzugsweise mindestens 10⁷ cfu/g, idealerweise mindestens 5x10⁷ cfu/g.

9. Symbiotische Zusammensetzung nach Anspruch 7 oder 8, wobei die probiotischen Mikroorganismen ausgewählt sind aus Bakterien oder Hefen der Gattungen *Bifidobacterium, Lactobacillus, Lactococcus, Streptococcus, Pediococcus*, *Enterococcus, Propionibacterium, Saccharomyces, Kluyveromyces* und deren Gemischen, vorzugsweise aus den Arten *Bifidobacterium longum, Lactobacillus casei, Lactobacillus rhamnosus, Lactobacillus helveticus und Saccharomyces torula.*

10. Nahrungsmittel- oder Reformprodukt, welches das präbiotische Produkt nach einem der Ansprüche 1 bis 6 umfasst oder die symbiotische Zusammensetzung nach einem der Ansprüche 7 bis 9.

11. Kosmetikprodukt, welches das präbiotische Produkt nach einem der Ansprüche 1 bis 6 umfasst oder die symbiotische Zusammensetzung nach einem der Ansprüche 7 bis 9.

12. Arzneimittel, welches das präbiotische Produkt nach einem der Ansprüche 1 bis 6 umfasst oder die symbiotische Zusammensetzung nach einem der Ansprüche 7 bis 9 in Form von Tabletten, Kapseln, Pulver oder Granula.

13. Verfahren zur Herstellung eines präbiotischen Produkts nach einem der Ansprüche 1 bis 6, das einen Schritt des Mischens von Melasse mit der Polyosidzusammensetzung umfasst, um ein Vorgemisch zu erhalten, daraufhin einen Schritt des Trocknens des Vorgemischs.

14. Verfahren zur Herstellung eines symbiotischen Produkts nach einem der Ansprüche 7 bis 9, das Folgendes umfasst:
- einen Schritt des Mischens von Melasse mit der Polyosidzusammensetzung, um ein Vorgemisch zu erhalten, daraufhin einen Schritt des Trocknens des Vorgemischs, daraufhin einen Schritt des Mischens des getrockneten Vorgemischs mit der Zusammensetzung von probiotischen Mikroorganismen; oder
- einen Schritt des Mischens von Melasse mit der Polyosidzusammensetzung und der Zusammensetzung von probiotischen Mikroorganismen, um ein Vorgemisch zu erhalten, daraufhin einen Schritt des Trocknens des Vorgemischs.

15. Verfahren nach Anspruch 13 oder 14, wobei der Schritt des Trocknens mittels eines Vakuum-Trockenschranks, durch Lyophilisierung, durch Mikrowellenbestrahlung, mittels eines Wirbelbetts oder durch Atomisierung durchgeführt wird und vorzugsweise durch Atomisierung; und/oder wobei der Gehalt an Trockenmaterial des Vorgemischs vor dem Trocknen durch Hinzufügen von Wasser gesenkt wird, vorzugsweise bis zu einem Wert von 30 bis 60 Gew.%, besonders bevorzugt 40 bis 50 Gew.%.

## Claims

1. Prebiotic product comprising:
- dried molasses, in which the dried molasses have a dry matter content greater than or equal to 80%, in a mixture with
- a composition of polysaccharides,
in which the composition of polysaccharides has a fibre content greater than or equal to 80%.

2. Prebiotic product according to claim 1, in which the composition of polysaccharides is a composition of dextrin or maltodextrin, preferably a composition of dextrin derived from maize starch.

3. Prebiotic product according to claim 1 or 2, comprising a total proportion by weight of water less than or equal to 6%, preferably less than or equal to 5%, more particularly preferably less than 4%.

4. Prebiotic product according to one of claims 1 to 3, in which the composition of polysaccharides has:
- a content of monosaccharides and disaccharides less than or equal to 5%, preferably less than or equal to 2%, more particularly preferably less than or equal to 1% and ideally less than or equal to 0.5%; and/or
- a weight-average molecular mass from 4000 to 7000 daltons, preferably from 4500 to 6000 daltons and more particularly preferably from 4800 to 5200 daltons; and/or
- a viscosity, at 60% in water at 20°C, from 1000 to 2500 mPa.s, preferably from 1250 to 2000 mPa.s and more particularly preferably from 1400 to 1600 mPa.s; and/or
- from 45 to 55% of (1,4) glycosidic bonds, from 25 to 35% of (1,6) glycosidic bonds, from 5 to 15% of (1,2) glycosidic bonds and from 5 to 15% of (1,3) glycosidic bonds.

5. Prebiotic product according to one of claims 1 to 4, comprising, in proportions by weight: from 50 to 80% of the composition of polysaccharides and from 20 to 50% of dried molasses, preferably from 60 to 70% of the composition of polysaccharides and from 30 to 40% of dried molasses.

6. Prebiotic product according to one of claims 1 to 5, in which the molasses are chosen from the molasses produced from cane, beet, fruits, cereals and mixtures thereof, and preferably from the molasses produced from cane, beet and mixtures thereof, and more particularly preferably is cane molasses.

7. Symbiotic composition comprising the prebiotic product according to one of claims 1 to 6, in a mixture with a composition of probiotic micro-organisms, preferably living, dehydrated and encapsulated micro-organisms.

8. Symbiotic composition according to claim 7, comprising in proportions by weight
- from 10 to 50% of dried molasses, preferably from 15 to 45%, more particularly preferably from 20 to 40%;
- from 15 to 70% of the composition of polysaccharides, preferably from 20 to 65%, more particularly preferably from 35 to 60%; and
- from 4 to 50% of the composition of probiotic micro-organisms, preferably from 10 to 40%, more particularly preferably from 20 to 30%;
and/or comprising a quantity of living probiotic micro-organisms of at least 10⁵ cfu/g, preferably at least 10⁷ cfu/g, ideally at least 5 x 10⁷ cfu/g.

9. Symbiotic composition according to claim 7 or 8, in which the probiotic micro-organisms are chosen from the bacteria or yeasts of the *Bifidobacterium, Lactobacillus, Lactococcus, Streptococcus, Pediococcus, Enterococcus, Propionibacterium, Saccharomyces, Kluyveromyces* genera and mixtures thereof, and preferably from the *Bifidobacterium longum, Lactobacillus casei, Lactobacillus rhamnosus, Lactobacillus helveticus* and *Saccharomyces torula* species.

10. Food or dietetic product comprising the prebiotic product according to one of claims 1 to 6, or the symbiotic composition according to one of claims 7 to 9.

11. Cosmetic product comprising the prebiotic product according to one of claims 1 to 6, or the symbiotic composition according to one of claims 7 to 9.

12. Medicament comprising the prebiotic product according to one of claims 1 to 6, or the symbiotic composition according to one of claims 7 to 9, in the form of tablets, gelatin capsules, powder or granules.

13. Process for manufacturing a prebiotic product according to one of claims 1 to 6, comprising a step of mixing the molasses with the composition of polysaccharides in order to obtain a premix, then a step of drying the premix.

14. Process for manufacturing a symbiotic composition according to one of claims 7 to 9, comprising:
- a step of mixing the molasses with the composition of polysaccharides in order to obtain a premix, then a step of drying the premix, then a step of mixing the dried premix with the composition of probiotic microorganisms; or
- a step of mixing the molasses with the polysaccharide composition and the composition of probiotic microorganisms in order to obtain a premix, then a step of drying the premix.

15. Process according to claim 13 or 14, in which the drying step is carried out using an oven under vacuum, by lyophilization, by microwave radiation, using a fluidized bed or by atomization, and preferably by atomization; and/or in which the dry matter content of the premix before drying is reduced by the addition of water, preferably up to a value of 30 a 60%, more particularly preferably from 40 to 50% by weight.
